# EUROPEAN PATENT SPECIFICATION

(11) **EP 1 585 502 B1**
(45) Date of publication and mention of the grant of the patent: **29.06.2011**
(21) Application number: 03811260.3
(22) Date of filing: 12.11.2003
(51) Int. Cl.: A61K 9/26, A61K 9/19, A61K 31/436, A61K 9/51

(54) **FAST-DISINTEGRATING SOLID DOSAGE FORMS BEING NOT FRIABLE AND COMPRISING PULLULAN**
RASCH ZERFALLENDE FESTE DARREICHUNGSFORMEN MIT NICHTBRÖSELIGER KONSISTENZ UND PULLULAN
FORMES POSOLOGIQUES SOLIDES A DESINTEGRATION RAPIDE NON FRIABLES ET COMPRENANT DU PULLULANE

(30) Priority: 12.11.2002 US 425264 P
(43) Date of publication of application: 19.10.2005
(73) Proprietor: Elan Pharma International Limited, Athlone, County Westmeath (IE)
(72) Inventor: PRUITT, John, D., Collegeville, PA 19426 (US); HOVEY, Douglas, C., Gilbertsville, PA 19525 (US); RYDE, Tuula, A., Malvern, PA 19355 (US); BOSCH, H., William, Bryn Mawr, PA 19010 (US); LEE, Robert, W., Boyertown, PA 19512-7547 (US)
(74) Representative: Ryan, Anne Mary
(86) International application number: PCT/US2003/035915
(87) International publication number: WO 2004/043440

(56) References cited:
- EP-A- 1 022 021
- WO-A-00/50013
- WO-A-01/70194
- WO-A-2004/012720
- US-A- 5 411 945
- US-A1- 2001 022 964
- US-B1- 6 413 541

## Description

The present invention relates to solid dosage forms of active agents comprising pullulan and having remarkably low Inability. The active agent is nanoparticulate and soluble or poorly soluble in water.

### BACKGROUND OF THE INVENTION

The present invention encompasses all dosage forms, such as controlled release formulations, fast melt formulations, aerosol formulations, lyophilized formulations, tablets, solid lozenges, capsules, powders, etc. In a preferred embodiment, the solid dosage form is a rapidly disintegrating or dissolving dosage form, *i.e.,* a fast melt dosage form.

### A. Background Regarding Rapidly Dissolving Compositions

Current manufacturers of rapidly disintegrating or dissolving solid dose oral formulations include, for example, Cima Labs, Fuisz Technologies Ltd., Prographarm, R.P. Scherer, and Yamanouchi-Shaklee. All of these manufacturers market different types of rapidly dissolving solid oral dosage forms.

Cima Labs markets OraSolv^{®}, which is an effervescent direct compression tablet having an oral dissolution time of five to thirty seconds, and DuraSolv^{®}, which is a direct compression tablet having a taste-masked active agent and an oral dissolution time of 15 to 45 seconds. The OraSolv^{®} formulation in particular has a very high degree of friability. Cima's U.S. Patent No. 5,607,697, for "Taste Masking Microparticles for Oral Dosage Forms," describes a solid dosage form consisting of coated microparticles that disintegrate in the mouth. The microparticle core has a pharmaceutical active agent and one or more sweet-tasting compounds having a negative heat of solution selected from mannitol, sorbitol, a mixture of an artificial sweetener and menthol, a mixture of sugar and menthol, and methyl salicylate. The microparticle core is coated, at least partially, with a material that retards dissolution in the mouth and masks the taste of the pharmaceutical active agent. The microparticles are then compressed to form a tablet. Other excipients can also be added to the tablet formulation.

WO 98/46215 for "Rapidly Dissolving Robust Dosage Form," assigned to Cima Labs, is directed to a hard, compressed, fast melt formulation having an active ingredient and a matrix of at least a non-direct compression filler and lubricant. A non-direct compression filler is typically not free-flowing, in contrast to a direct compression (DC grade) filler, and usually requires additionally processing to form free-flowing granules.

Cima also has U.S. patents and international patent applications directed to effervescent dosage forms (U.S. Patent Nos. 5,503,846, 5,223,264, and 5,178,878) and tableting aids for rapidly dissolving dosage forms (U.S. Patent Nos. 5,401,513 and 5,219,574), and rapidly dissolving dosage forms for water soluble drugs (WO 98/14179 for "Taste-Masked Microcapsule Composition and Methods of Manufacture").

Fuisz Technologies, now part of BioVail, markets Flash Dose^{®}, which is a direct compression tablet containing a processed excipient called Shearform^{®}. Shearform^{®} is a cotton candy-like substance of mixed polysaccharides converted to amorphous fibers. U.S. patents describing this technology include U.S. Patent No. 5,871,781 for "Apparatus for Making Rapidly Dissolving Dosage Units;" U.S. Patent No. 5,869,098 for "Fast-Dissolving Comestible Units Formed Under High-Speed/HighPressure Conditions;" U.S. Patent Nos. 5,866,163, 5,851,553, and 5,622,719, all for "Process and Apparatus for Making Rapidly Dissolving Dosage Units and Product Therefrom;" U.S. Patent No. 5,567,439 for "Delivery of Controlled-Release Systems;" and U.S. Patent No. 5,587,172 for "Process for Forming Quickly Dispersing Comestible Unit and Product Therefrom."

Prographarm markets Flashtab^{®}, which is a fast melt tablet having a disintegrating agent such as carboxymethyl cellulose, a swelling agent such as a modified starch, and a taste-masked active agent. The tablets have an oral disintegration time of under one minute (U.S. Patent No. 5,464,632).

R.P. Scherer markets Zydis^{®}, which is a freeze-dried tablet having an oral dissolution time of 2 to 5 seconds. Lyophilized tablets can be costly to manufacture and difficult to package because of the tablets' sensitivity to moisture and temperature. U.S. Patent No. 4,642,903 (R.P. Scherer Corp.) refers to a fast melt dosage formulation prepared by dispersing a gas throughout a solution or suspension to be freeze-dried. U.S. Patent No. 5,188,825 (R.P. Scherer Corp.) refers to freeze-dried dosage forms prepared by bonding or complexing a water-soluble active agent to or with an ion exchange resin to form a substantially water insoluble complex, which is then mixed with an appropriate carrier and freeze dried. U.S. Patent No. 5,631,023 (R. P. Scherer Corp.) refers to freeze-dried drug dosage forms made by adding xanthan gum to a suspension of gelatin and active agent. U.S. Patent No. 5,827,541 (R.P. Scherer Corp.) discloses a process for preparing solid pharmaceutical dosage forms of hydrophobic substances. The process involves freeze-drying a dispersion containing a hydrophobic active ingredient and a surfactant in a non-aqueous phase; and a carrier material in an aqueous phase.

Yamanouchi-Shaklee markets Wowtab^{®}, which is a tablet having a combination of a low moldability and a high moldability saccharide. U.S. Patents covering this technology include U.S. Patent No. 5,576,014 for "Intrabuccally Dissolving Compressed Moldings and Production Process Thereof," and U.S. Patent No. 5,446,464 for "Intrabuccally Disintegrating Preparation and Production Thereof."

Other companies owning rapidly dissolving technology include Janssen Pharmaceutica. U.S. patents assigned to Janssen describe rapidly dissolving tablets having two polypeptide (or gelatin) components and a bulking agent; wherein the two components have a net charge of the same sign, and the first component is more soluble in aqueous solution than the second component. *See* U.S. Patent No. 5,807,576 for "Rapidly Dissolving Tablet;" U.S. Patent No. 5,635,210 for "Method of Making a Rapidly Dissolving Tablet;" U.S. Patent No. 5,595,761 for "Particulate Support Matrix for Making a Rapidly Dissolving Tablet;" U.S. Patent No. 5,587,180 for "Process for Making a Particulate Support Matrix for Making a Rapidly Dissolving Tablet;" and U.S. Patent No. 5,776,491 for "Rapidly Dissolving Dosage Form."

Eurand America, Inc. has U.S. patents directed to a rapidly dissolving effervescent composition having a mixture of sodium bicarbonate, citric acid, and ethylcellulose (U.S. Patent Nos. 5,639,475 and 5,709,886).

L.A.B. Pharmaceutical Research owns U.S. patents directed to effervescent-based rapidly dissolving formulations having an effervescent couple of an effervescent acid and an effervescent base (U.S. Patent Nos. 5,807,578 and 5,807,577).

Schering Corporation has technology relating to buccal tablets having an active agent, an excipient (which can be a surfactant) or at least one of sucrose, lactose, or sorbitol, and either magnesium stearate or sodium dodecyl sulfate (U.S. Patent Nos. 5,112,616 and 5,073,374).

Laboratoire L. LaFon owns technology directed to conventional dosage forms made by lyophilization of an oil-in-water emulsion in which at least one of the two phases contains a surfactant (U.S. Patent No. 4,616,047). For this type of formulation, the active ingredient is maintained in a frozen suspension state and is tableted without micronization or compression, as such processes could damage the active agent.

Takeda Chemicals Inc., Ltd. owns technology directed to a method of making a fast dissolving tablet in which an active agent and a moistened, soluble carbohydrate are compression molded into a tablet, followed by drying of the tablets.

Finally, Elan Pharma International Ltd.'s U.S. Patent No. 6,316,029 describes rapidly dissolving dosage forms comprising poorly soluble nanoparticulate active agents. The present invention is an improvement over the invention of U.S. Patent No. 6,316,029 as this patent does not teach dosage forms comprising pullulan.

EP1022021 A, US5411945 A, WO005001 A, US6413541 B, WO0170194 A or US 2001022964 do not describe the particle size range of present claim 1.

### B. Background Regarding Pullulan

Pullulan (CAS Reg. No. 9057-02-7) is an extracellular polysaccharide excreted by the fungus *Aureobasidium pullulans.* It is an alpha-D-glucan consisting predominantly of repeating maltotrioses (*i.e*., glucose units) linked by alpha-1,6-glucosidic bonds. This repeating sequence forms a stair-step-type structure. Occasional maltotetrose units are distributed randomly throughout the polymer. Molecular weights for pullulan range from 8,000 to 2,000,000 daltons depending on the growth conditions of the organism. Pullulan is soluble in hot and cold water and is generally insoluble in organic solvents. Pullulan is non-hygroscopic and non-reducing; it decomposes at 250 to 280 degrees C.

The glucose units of pullulan are polymerized in such a way as to make the compound viscous and impermeable to oxygen. The viscosity of water solutions of pullulan is proportional to the molecular weight of the pullulan. Water solutions are stable and do not form gels. Pullulan readily forms a film, which is thermally stable, anti-static, and elastic. Pullulan has adhesive properties and is directly compressible under heat with moisture. *See* Agency Response Letter GRAS Notice No. GRN 000099 (Aug. 1, 2002), htlp://vm.cfsan.fda.gov/~rdb/opa-g099.html; and the Dictionary of Biology (Oxford University Press, 2000), http://www.xrefer.com/entry/463045.

Pullulan is used in adhesives, food packaging, and molded articles. In the food industry, pullulan can be used as a thickener, binding agent, as well as a food ingredient. Its physical properties make it suitable as a wrapping, packaging, and sealing material. It can also be laminated in tea bags and used to preserve freshness of eggs and egg products. In another application, pullulan can be used as a composition of industrial products such as textiles, paints, cosmetics, adhesives, photography, tobacco products, etc. *See* http://www.mardi.my/ver2/rangkaian inovasi/fed batch.html (2001)

U.S. Patent No. 5,518,902, for "High Pullulan Content Product, and its Preparation and Uses," to Ozaki et al. refers to a high pullulan content product having an average molecular weight of less than 250,000. The product is prepared by continuously cultivating a microorganism capable of producing pullulan in a nutrient culture medium containing a 10-20 w/v % saccharide while controlling the viscosity of the nutrient culture medium to a level below 30 cp. This reference further discloses that the high pullulan content product can be advantageously used in a variety of fields such as viscosity-imparting agent, coating agent, adhesive, formed product, food product, cosmetic, pharmaceutical, and material for agriculture, forestry, stock raising and paper processing, as well as for mining and manufacturing industries. This reference does not teach a solid dosage form comprising pullulan and having a low friability.

### C. Background Regarding Nanoparticulate Compositions

Nanoparticulate compositions, first described in U.S. Patent No. 5,145,684 ("the '684 patent"), are particles consisting of a poorly soluble active agent having adsorbed onto the surface thereof a non-crosslinked surface stabilizer. The `684 patent also describes methods of making such nanoparticulate compositions. Nanoparticulate compositions are desirable because with a decrease in particle size, and a consequent increase in surface area, a composition is rapidly dissolved and absorbed following administration. The `684 patent does not teach or suggest nanoparticulate compositions comprising pullulan.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastorointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;'' 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for 0"Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" and 6,432,381 for "Methods for Targeting Drug Delivery to the Upper and/or Lower Gastrointestinal Tract," all of which are specifically incorporated by reference. In addition, U.S. Patent Application No. 20020012675 A1, published on January 31, 2002, for "Controlled Release Nanoparticulate Compositions," describes nanoparticulate compositions, and is specifically incorporated by reference.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." None of these references relates to a nanoparticulate fast melt composition comprising pullulan.

There is a need in the art for improved solid dosage forms having low friability. The present invention satisfies this need.

### SUMMARY OF THE INVENTION

This invention is directed to the surprising and unexpected discovery of new solid dosage forms of active agents comprising pullulan and having a remarkably low friability of less than about 1%. Additional pharmaceutically acceptable excipients can also be added to the composition. The present invention encompasses all solid dosage forms, such as controlled release formulations, fast melt formulations, aerosol formulations, lyophilized formulations, tablets,-solid lozenges, capsules, powders, etc.

In the present invention, the active agent has a nanoparticulate particle size, meaning that the active agent has an effective average particle size of less than about 2 microns prior to formulation into a solid dosage form. The nanoparticulate active agent can be water-soluble or poorly water soluble. If the nanoparticulate active agent is water-soluble it can be rendered poorly water-soluble by complexing or another pharmaceutically acceptable means. In addition, the nanoparticulate active agent can have one or more surface stabilizers adsorbed onto the surface of the active agent.

A first embodiment of the invention encompasses solid dosage forms of active agents having highly toxic and/or highly potent properties.

In a second embodiment, the solid dosage form additionally comprises a pharmaceutically acceptable sugar. A solid dosage form according to this embodiment comprises: (1) pullulan, (2) at least one active agent, which is nanoparticulate, and either water-soluble or poorly water-soluble; and (3) one or more pharmaceutically acceptable sugars. As the active agent has a nanoparticulate particle size prior to inclusion in the dosage form, then the solid dosage form may also comprise one or more surface stabilizers adsorbed to the surface of the nanoparticulate active agent.

In a third embodiment, the solid dosage form additionally comprises a plasticizer. A solid dosage form according to this embodiment comprises: (1) pullulan, (2) at least one an active agent, which is nanoparticulate, and either water-soluble or poorly water-soluble; and (3) one or more pharmaceutically acceptable plasticizers. In addition, the solid dosage form may also comprise one or more pharmaceutically acceptable sugars. As the active agent has a nanoparticulate particle size prior to inclusion in the dosage form, then the solid dosage form may also comprise one or more surface stabilizers adsorbed to the surface of the nanoparticulate active agent.

In a fourth embodiment, the solid dosage form is a fast melt solid dosage form. The fast melt solid dosage form comprises: (1) pullulan and (2) an active agent, which is nanoparticulate, and either water-soluble or poorly water-soluble. The solid dosage form may also comprise: (3) one or more pharmaceutically acceptable sugars and/or (4) one or more pharmaceutically acceptable plasticizers. Additional pharmaceutically acceptable excipients can also be added to the composition. As the active agent has a nanoparticulate particle size prior to inclusion in the dosage form, then the solid dosage form may also comprise at least one surface stabilizer. In such a fast melt solid dosage form, the solid dose matrix surrounding the active agent disintegrates or dissolves upon contact with saliva, thereby presenting the active agent for absorption. Such a rapidly disintegrating or dissolving solid dosage form according to the invention provides an unexpectedly fast onset of therapeutic activity, substantially complete disintegration or dissolution of the formulation in less than about 4 minutes, and extremely low friability.

Most surprising is the discovery that the fast melt solid dosage forms of the invention have a very low friability. This is significant as prior art fast melt solid dosage forms have a high friability, resulting in additional manufacturing and packaging costs.

In a fifth embodiment of the invention there is provided a method of preparing the solid dosage forms of the invention. The method comprises: (1) providing an active agent composition, wherein the active agent is
nanoparticulate, and either water soluble or poorly water-soluble; (2) combining the active agent composition with pullulan; and (3) forming a solid dosage form having a friability of less than about 1%, utilizing a pharmaceutically acceptable method. The method can additionally comprise adding: (1) one or more surface stabilizers, as the active agent has a nanoparticulate particle size prior to inclusion in the dosage form; (2) one or more pharmaceutically acceptable sugars; and/or (3) one or more pharmaceutically acceptable plasticizers. Additional pharmaceutically acceptable excipients can also be added to the composition.

In a sixth embodiment of the invention there is provided a method of treating a subject, including a mammal or a human, with a solid dosage form comprising pullulan according to the invention.

It is to be understood that both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPTION OF THE INVENTION

### A. Solid Dosage Forms

This invention is directed to the surprising and unexpected discovery of new solid dosage forms of active agents Comprising pullulan and having remarkably low friability. The dosage forms of the invention comprises: (1) pullulan and (2) one or more active agents, which are nanoparticulate prior to inclusion in the solid dosage form, and either water-soluble or poorly water soluble. The present invention encompasses all solid dosage forms, including but not limited to controlled release formulations, fast melt formulations, aerosol formulations, lyophilized formulations, tablets, solid lozenges, capsules, powders, etc. In sum, the solid dosage form of the invention can be any pharmaceutically acceptable solid dosage form.

In a preferred embodiment, the solid dosage form is a rapidly disintegrating or dissolving dosage form, *i.e*., a fast melt dosage form.

One problem encountered with prior art solid dosage forms was that the dosage forms often exhibited a high friability. Friability is a physical parameter of a solid dosage form; it basically refers to the dosage form's "robustness." Dosage forms having a high friability will rapidly dissolve or disintegrate. However, an optimum solid dosage form will rapidly dissolve or disintegrate *and* have a low level of friability. The present invention provides this combination of desirable traits. Specifically, the pullulan-comprising solid dosage forms of the invention have a surprisingly fast disintegration and dissolution profile, with some tablets dissolving in a few seconds (see the following examples). More surprising is that these same tablets have a friability of less than about 1%, meaning that the tablets meet the United States Pharmacopeia standard for tablet friability (which requires a friability of less than 1%).

For the solid dosage forms of the invention preferably have a friability of less than about 1%, less than about 0.9%, less than about 0.8%, less than about 0.7%, less than about 0.6%, less than about 0.5%, less than about 0.4%, less than about 0.3%, or less than about 0.2%.

"Nanoparticulate" is defined as an active agent having an effective average particle size prior to inclusion in the solid dosage form of less than about 2 microns, and "micron-sized" is defined as having an effective average particle size of greater than about 2 microns prior to inclusion in the solid dosage form.

A micron-sized active agent can be water-soluble or poorly water-soluble. In addition, a nanoparticulate active agent can be water-soluble or poorly water-soluble. If the nanoparticulate active agent is water-soluble, then if desired it can be rendered poorly water-soluble by, for example, complexing the active agent with a non-soluble compound or utilizing any other pharmaceutically acceptable means. By "poorly soluble" it is meant that the active agent has a solubility in water of less than about 30 mg/ml, less than about 10 mg/ml, or less than about 1 mg/ml at ambient temperature and pressure.

As the active agent has a nanoparticulate particle size prior to inclusion in the solid dosage form, then the solid dosage form can additionally comprise one or more surface stabilizers, which are adsorbed to the surface of the active agent prior to inclusion of the active agent in the solid dosage form.

In one embodiment of the invention, the solid dosage form additionally comprises a pharmaceutically acceptable sugar. A solid dosage form according to this embodiment comprises: (1) pullulan, (2) at least one active agent, which is
nanoparticulate, and either water-soluble or poorly water-soluble; and (3) one or more pharmaceutically acceptable sugars. As the active agent has a nanoparticulate particle size prior to inclusion in the solid dosage form, then the solid dosage form may also comprise one or more surface stabilizers, which are adsorbed to the surface of the nanoparticulate active agent prior to inclusion in the solid dosage form. Exemplary useful pharmaceutically acceptable sugars are provided below.

In another embodiment, the solid dosage form additionally comprises a plasticizer. A solid dosage form according to this embodiment comprises: (1) pullulan, (2) at least one active agent, which is nanoparticulate, and either water-soluble or poorly water-soluble; and (3) one or more pharmaceutically acceptable plasticizers. In addition, the solid dosage form may also comprise one or more pharmaceutically acceptable sugars. As the active agent has a Nanoparticulate particle size prior to inclusion in the solid dosage form, then the solid dosage form may also comprise one or more surface stabilizers, which are adsorbed to the surface of the nanoparticulate active agent prior to inclusion in the solid dosage form. Exemplary useful pharmaceutically acceptable plasticizers are provided below.

The solid dosage forms of the invention can be formulated to mask the unpleasant taste of an active agent Such taste masking can be accomplished, for example, by the addition of one or more sweet tasting excipients, by coating the active agent with a sweet tasting excipient, and/or by coating a dosage form of an active agent and pullulan with a sweet tasting excipient.

Another embodiment of the invention encompasses solid dosage forms of active agents having highly toxic or potent properties. Highly toxic compounds include those which are known or thought to be cytotoxic, teratogenic, mutagenic, immunosuppressant, or have negative pharmacological effects. Compounds having potent properties are those which induce pharmacological effects at doses less than about 10mg in normal human subjects. A solid dosage form according to this embodiment comprises: (1) pullulan and (2) one or more active agents having highly toxic and/or portent properties, in which the active agent is nanoparticulate, and either water-soluble or poorly water-soluble, and optionally (3) one or more surface stabilizers adsorbed to the surface of the nanoparticulate active agent prior to inclusion of the active agent in the dosage form, (4) one or more pharmaceutically acceptable sugars, and/or (5) one or more pharmaceutically acceptable plasticizers.

The solid dosage forms of the invention, comprising for example highly potent and/or toxic active agents, can be made in a dust-less process. This is significant as conventional methods of making solid dosage forms inherently produce a dust or fine powder of the solid dosage form material. If such a material comprises an active agent having highly toxic or potent properties, then extensive and expensive safety procedures, along with containment apparatus, is required. Solid dosage forms made according to one method of the invention avoid this problem. The method comprises:
(1) providing a dispersion or solution of an active agent, wherein the active agent is nanoparticulate, and either water soluble or poorly water-soluble;
(2) providing a solution comprising pullulan; (3) combining the active agent dispersion or solution with the pullulan solution; and (4) formulating the mixture of the dispersion/solution or solution/solution into a solid dosage form via any pharmaceutically acceptable method, such as by lyophilization. This method is sample, efficient, and can be adapted to almost any active agent This makes the method particularly useful for generating tablets for clinical trials (or for any other purpose).

In yet another embodiment, the solid dosage form is a fast melt solid dosage form. Rapidly disintegrating or dissolving dosage forms, also known as fast dissolve, fast or rapid melt, and quick disintegrating dosage forms, dissolve or disintegrate rapidly in the patient's mouth without chewing or the need for water within a short time frame. The fast melt solid dosage form comprises: (1) pullulan and (2) at least one active agent, which is nanoparticulate, and either water-soluble or poorly water-soluble. The solid dosage form may also comprise: (3) one or more pharmaceutically acceptable sugars and/or (4) one or more pharmaceutically acceptable plasticizers. Additional pharmaceutically acceptable excipients can also be added to the composition. As the active agent has a nanoparticulate particle size prior to inclusion in the solid dosage form, then the solid dosage form may also comprise at least one surface stabilizer, which is adsorbed to the surface of the nanoparticulate active agent prior to inclusion in the solid dosage form. The solid dosage form has an unexpectedly fast onset of therapeutic activity, substantially complete disintegration or dissolution of the formulation in less than about 4 minutes, and extremely low friability

For the fast melt solid dosage forms of the invention, the solid dose matrix surrounding the active agent disintegrates or dissolves upon contact with saliva, thereby presenting the active agent for absorption. Thus, such a rapidly disintegrating or dissolving solid dosage form according to the invention provides: (1) rapid presentation of the active agent as a result of the rapid disintegration, (2) rapid dissolution of the active agent in the oral cavity, particularly if the active agent has a nanoparticulate particle size, and (3) low friability of the solid dosage form, which results in dramatically improved manufacturing and packaging costs.

A fast melt solid dosage form according to the invention has a disintegration time of less than about 4 minutes upon addition to an aqueous medium. In other embodiments of the invention the fast melt solid dosage forms have a disintegration or dissolution time upon addition to an aqueous medium of less than about 3.5 minutes, less than about 3 minutes, less than about 2.5 minutes, less than about 2 minutes, less than about 90 seconds, less than about 60 seconds, less than about 45 seconds, less than about 30 seconds, less than about 20 seconds, less than about 15 seconds, less than about 10 seconds, or less than about 5 seconds.

Most surprising is the discovery that the fast melt solid dosage forms of the invention have a very low friability. This is significant as prior art fast melt solid dosage forms have a high level of friability, resulting in additional manufacturing and packaging costs. For example, traditional blister packaging cannot be utilized for solid dosage forms having a high degree of friability, as when the consumer "pushes" the dosage form out of the sealed compartment (typically through a foil sealer), such a dosage form would disintegrate into a powder. Moreover, in manufacturing solid dosage forms having a high degree of friability, a greater percentage of the material is lost as waste, such as in broken or disintegrated tablets. Finally, tablets having a high degree of friability can be problematic in manufacturing when the active agent in the solid dosage form is highly toxic or potent, as the high level of friability increases the risk of accidental exposure.

This combination of rapid disintegration, rapid dissolution, and low friability reduces the delay in the onset of therapeutic action associated with prior known rapidly dissolving dosage forms of active agents. Further, the opportunity for buccal absorption of the active agent is enhanced with the present invention. Yet another advantage of the solid dosage forms of the invention is that the use of nanoparticulate active agent particles eliminates or minimizes the feeling of grittiness found with prior art fast melt formulations of poorly soluble drugs.

Because of their ease of administration, fast melt solid dosage forms are particularly useful for the specific needs of pediatrics, geriatrics, and patients with dysphagia. Fast melt solid dosage forms can be beneficial because of their ease of administration, convenience, and patient-friendly nature. It is estimated that 35% to 50% of the population finds it difficult to swallow tablets and hard gelatin capsules, particularly pediatric and geriatric patients. Fast melt solid dosage forms eliminate the need to swallow a tablet or capsule. Moreover, fast melt solid dosage forms do not require the addition of water or chewing.

One advantage typically associated with fast melt solid dosage forms is a reduction of the time lag between administration of a dose and the physical presentation of the active agent. This lag time is usually associated with the break up of the dosage form and the distribution of the active agent thereafter. A second advantage of fast melt solid dosage forms is that the rapid presentation of the active agent in the mouth upon administration may facilitate buccal absorption of the active agent directly into the blood stream, thus reducing the first pass effect of the liver on the overall bioavailability of active agent from a unit dose. This second advantage is dramatically enhanced for the fast melt solid dosage forms of the invention comprising nanoparticulate active agents, as the nanoparticulate size of the active agent enables rapid dissolution in the oral cavity.

### 1. Active Agents

The active agent may be present either substantially in the form of one optically pure enantiomer or as a mixture, racemic or otherwise, of enantiomers. In addition, the active agent exists as a discrete, crystalline phase, as an amorphous phase, a semi-crystalline phase, a semi-amorphous phase, or a combination thereof. The active agent can have a micron-sized particle size or a nanoparticulate particle size, and the active agent is either water-soluble or poorly water-soluble.

Exemplary active agents can be therapeutic or diagnostic agents, collectively referred to as "drugs". A therapeutic agent can be a pharmaceutical agent, including biologics such as proteins, peptides, and nucleotides, or a diagnostic agent, such as a contrast agent, including x-ray contrast agents.

The active agent can be selected from a variety of known classes of drugs, including, for example, COX-2 inhibitors, retinoids, anticancer agents, NSAIDS, proteins, peptides, nucleotides, anti-obesity drugs, nutraceuticals, corticosteroids, elastase inhibitors, analgesics, anti-fungals, oncology therapies, anti-emetics, analgesic, cardiovascular agents, anti-inflammatory agents, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (*e.g*.,hypnotics and neuroleptics), astringents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio- pharmaceuticals, sex hormones (including steroids), anti-allergic agents, stimulants and anoretics, sympathomimetics, thyroid agents, vasodilators, xanthines, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.

Exemplary nutraceuticals and dietary supplements are disclosed, for example, in Roberts et al., Nutraceuticals: The Complete Encyclopedia of Supplements, Herbs, Vitamin, and Healing Foods (American Nutraceutical Association, 2001), which is specifically incorporated by reference. A nutraceutical or dietary supplement also known as phytochemicals or functional foods, is generally any one of a class of dietary supplements, vitamin, minerals, herbs, or healing foods that have medical or pharmaceutical effects non the body. Exemplary nutraceuticals or dietary supplements include, but are not limited to, folic acid, fatty acids (*e.g*., DHA and ARA), fruit and vegetable extracts, vitamin and mineral supplements, phosphatidylserine, lipoic acid, melatonin, glucosamine/chondroitin, Aloe Vera, Guggul, glutamine, amino acids (*e.g*., iso-leucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

The active agents are commercially available and/or can be prepared by techniques known in the art.

### 2. Surface Stabilizers for Nanoparticulate Active Agents

As the active agent has a nanoparticulate particle size prior to inclusion in the solid dosage form, then the active agent can have one or more surface stabilizers adsorbed to the surface of the nanoparticulate active agent.

Surface stabilizer useful herein physically adhere on the surface of the nanoparticulate active agent but do not chemically react with the active agent particles or itself. Individually adsorbed molecules of the surface stabilizer are essentially free of intermolecular cross-linkages.

Exemplary useful surface stabilizers include, but are not limited to, known organic and inorganic pharmaceutical excipients. Such excipients include various polymers, low molecular weight oligomers, natural products, and surfactants. Preferred surface stabilizers include nonionic and ionic surfactants, including anionic and cationic surfactants. Combinations of more than one surface stabilizer can be used in the invention.

Representative examples of surface stabilizers include hydroxypropyl methylcellulose, hydroxypropylcellulose, polyvinylpyrrolidone, random copolymers of vinyl pyrrolidone and vinyl acetate, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g*., the commercially available Tweens^{®} such as *e.g*., Tween 20^{®} and Tween 80^{®} (ICI Speciality Chemicals)); polyethylene glycols (*e.g*., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylinethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronics F68^{®} and F108^{®}, which are block copolymers of ethylene oxide and propylene oxide); poloxamines (*e.g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Dow); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂C(O)N(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quaternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride,-decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthyhnethyl ammonium chloride, trimethylammonium halide, alkyl-trimethylammonium salts and dialkyl-dimethylammonium salts, lauryl trimethyl ammonium chloride, ethoxylated alkyamidoakyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quaternized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Particularly preferred nonpolymeric primary stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quaternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference. The surface stabilizers are commercially available and/or can be prepared by techniques known in the art.

### 3. Pullulan

Pullulan (CAS Reg. No. 9057-02-7) is an extracellular linear homopolysaccharide of glucose excreted by the fungus *Aureobasidium pullulans.* It is an alpha-D-glucan consisting predominantly of repeating maltotrioses (i.e., glucose units) linked by alpha-1,6-glucosidic bonds, and has the following structure.

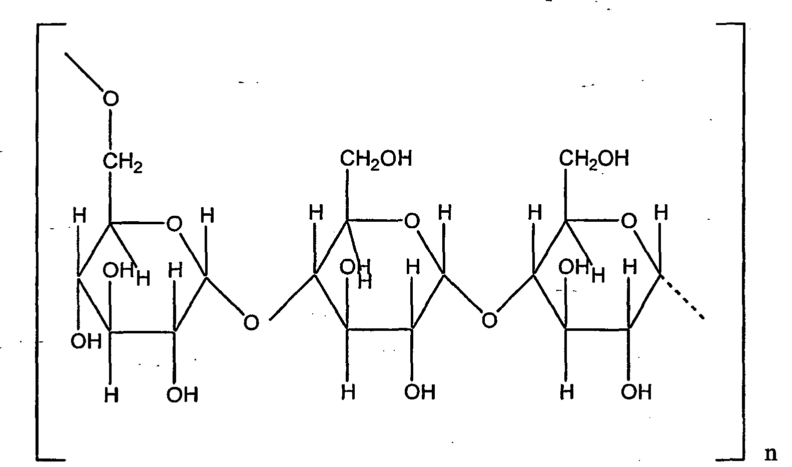

Pullulan's unique linkage pattern endows the compound with distinctive physical traits. Pullulan has adhesive properties and can be used to form fibers, compression moldings, and strong, oxygen-impermeable films. Pullulan is easily derivatized to control its solubility or provide reactive groups. Consequently, pullulan and its derivatives have numerous potential food, pharmaceutical, and industrial applications.

Pullulan is described in, for example Jakovljevi et al., "Fine Structural Analysis of the Fungal Polysaccharide Pullulan Elaborated by Aureobasidium pullulans, CH-1 Strain," J. Serb. Chem. Soc., 66(6):377-383 (2001); Agency Response Letter GRAS Notice No. GRN 000099 (Aug. 1,2002), http://vm.cfsan.fda.gov/-rdb/opa-g099.html: and the Dictionary of Biology (Oxford University Press, 2000), http://www.xrefer.com/entry/463045, which are specifically incorporated by reference.

### 4. Pharmaceutically Acceptable Sugars

Any pharmaceutically acceptable sugars can be employed in the solid dosage forms of the invention. Exemplary pharmaceutically acceptable sugars include, but are not limited to, sucrose, xylitol, lactose, mannitol, sorbitol, glucose, mannose, fructose, and trehalose.

### 5. Pharmaceutically Acceptable Plasticizers

Any pharmaceutically acceptable plasticizers can be employed in the solid dosage forms of the invention. Exemplary pharmaceutically acceptable plasticizers include, but are not limited to, glycerin, polyethylene glycol, propylene glycol, and sorbitol.

### 6. Other Pharmaceutical Excipients

Solid dosage forms according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicifized microcrystalline cellulose (SMCC).

Suitable lubricants, including agents that act on the flowability of the powder to be compressed, are colloidal silicon dioxide, such as Aerosil^{®} 200; talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for examples, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the acid component of the effervescent couple may be present.

### 7. Particles Size of the Active Agent

As used herein, particle size is determined on the basis of the weight average particle size as measured by conventional particle size measuring techniques well known to those spilled in the art. Such techniques include, for example, sedimentation field flow fractionation, photon correlation spectroscopy, light scattering, and disk centrifugation.

For "nanoparticulate active agents," by "an effective average particle size of less than about 2 microns" it is meant that at least 50% by weight of the active agent particles have a particle size less than the effective average, *i.e.,* less than about 2000 nm, 1900 nm, 1800 nm, *etc.,* when measured by the above-noted techniques. In other embodiments of the invention, at least about 70%, at least about 90%, at least about 95%, or at least about 99% of the active agent particles have a particle size less than the effective average, i.e., less than about 2000 nm, 1900 nm, 1800 nm, *etc.*

In addition, in other embodiments of the invention, the effective average particle size of the nanoparticulate active agent particles can be less than about 1900 nm, less than about 1800 nm, less than about 1700 nm, less than about 1600 mn, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, or less than about 50 nm.

In the present invention, the value for D50 of a nanoparticulate active agent composition is the particle size below which 50% of the active agent particles fall, by weight. Similarly, D90 and D99 are the particle sizes below which 90% and 99%, respectively, of the active agent particles fall, by weight.

### 8. Concentration of Active, Pullulan, Surface Stabilizer, Sugar, and Plasticizer

The relative amount of the at least one active agent and pullulan can vary widely. In addition, if the solid dosage form comprises one or more surface stabilizers, the optimal amount of the surface stabilizer(s) can depend, for example, upon the particular active agent selected, the equivalent hydrophilic lipophilic balance (HLB) of the active agent, the melting point, cloud point, and water solubility of the surface stabilizer, and the surface tension of water solutions of the stabilizer, *etc*. The active agent or pharmaceutically acceptable salt thereof may be present in any amount which is sufficient to elicit a therapeutic effect.

The concentration of the at least one active agent can vary from about 99.9% to about 0.01% by weight based on the total weight of the dry composition.

In the presence of one or more surface stabilizers, the concentration of the at least one active agent can vary from about 99.5% to about 0.001 %, from about 95% to about 0.1%, or from about 90% to about 0.5%, by weight, based on the total combined weight of the at least one active agent and the at least one surface stabilizer, not including other excipients.

The concentration of the at least one surface stabilizer can vary from about 0.0001 % to about 99.9%, from about 5% to about 90%, and from about 10% to about 70%, by weight, based on the total combined dry weight of the at least one active agent and the at least one surface stabilizer, not including other excipients.

The concentration of pullulan can vary from about 99.9% to about 0.1 % (w/w), about 85% to about 1% (w/w), about 60% to about 5% (w/w), and about 30% to about 10% by weight based on the total weight of the dry composition.

The concentration of the one or more pharmaceutically acceptable sugars can vary from about 1% to about 99% (w/w), based on the total weight of the dry composition.

The concentration of the one or more pharmaceutically acceptable plasticizers can vary from about 0.01% to about 70% (w/w), based on the total weight of the dry composition.

### B. Methods of Making Rapidly Disintegrating Solid Dose Active Agent Compositions Comprising Pullulan

In another aspect of the invention there is provided a methods of preparing solid dosage forms of active agents comprising pullulan. The method comprises: (1) providing an active agent compositions; (2) adding pullulan, and (3) forming a solid dosage form of the mixture of (1) and (2) for administration. Pharmaceutically acceptable excipients can also be added to the composition for administration. The method can additionally comprise adding: (1) one or more surface stabilizers, as the active agent has a nanoparticulate particle size prior to inclusion in the dosage form; (2) one or more pharmaceutically acceptable sugars; and/or (3) one or more pharmaceutically acceptable plasticizers. Any pharmaceutically acceptable method can be used for making the solid dosage forms of the invention.

The active agent is nanoparticulate, and can be water-soluble or poorly water-soluble.

One method of preparing solid dosage forms of the invention comprises:
(1) providing a dispersion or solution of an active agent, wherein the active agent is nanoparticulate, and either water soluble or poorly water-soluble;
(2) providing a solution comprising pullulan; (3) combining the active agent dispersion or solution with the pullulan solution; and (4) formulating the mixture of the dispersion/solution or solution/solution into a solid dosage form via any pharmaceutically acceptable method. A preferred method for step (4) for making fast melt compositions is lyophilization, although any pharmaceutically acceptable method can be used The method can additionally comprise adding to the dispersion or solutions: (1) one or more surface stabilizers, if the active agent has a nanoparticulate particle size prior to inclusion in the dosage form; (2) one or more pharmaceutically acceptable sugars; and/or (3) one or more pharmaceutically acceptable plasticizers. Additional pharmaceutically acceptable excipients can also be added to the compositions.

An example of this method as applied to a poorly water-soluble nanoparticulate active agent having a surface stabilizer adsorbed to the surface thereof is: (1) preparing a dispersion of the nanoparticulate active agent having at least one surface stabilizer adsorbed to the surface thereof; (2) preparing a solutions of pullulan, (3) combining the active agent dispersion with the pullulan solution; and (4) formulating the mixture of the dispersion/solution into a solid dosage form via any pharmaceutically acceptable method. A preferred method for making a fast melt solid dosage form is lyophilization.

This method is particularly preferred for active agents which are highly potent or toxic, as the method avoids generating any powder or dust of the active agent, such as that encountered with spray drying or spray granulating of an active agent. This is significant, as a manufacturing process which produces a powder of a highly toxic or potent compound requires extensive safety precautions and apparatus to avoid exposure problems. Such safety procedures and apparatus can be costly to implement Moreover, this method is simple, efficient, and can be adapted to almost any active agent.

Methods of making solid dosage forms are known in the art, and such methods can be employed in the present invention. For example, as described above dispersions or solutions of an active agent can be mixed with a pullulan solution, followed by lyophilization to make a solid dosage form. Alternatively, a powder or granulate of an active agent can be blended with a pullulan powder, followed by tableting or filling of capsules. A powder or granulate of a nanoparticulate active agent dispersion can be made by, for example, spray drying or spray granulating. For example, a nanoparticulate active agent dispersion can be spray granulated onto a pullulan powder. Exemplary methods of making powders from liquids comprising active agents are described below.

### 1. Methods of Making Nanoparticulate Active Agent Compositions

Methods of making nanoparticulate active agent compositions, which can comprise precipitation, microfluidization, mechanical means, such as grinding, or any other suitable size reduction process, are known in the art. For example, methods of making nanoparticulate compositions are described in the '684 patent and in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" U.S. Patent No. 5,665,331, for "Co-Microprecipitation of Nanoparticulate Pharmaceutical Agents with Crystal Growth Modifiers;" U.S. Patent No. 5,662,883, for "Co-Microprecipitation of Nanoparticulate Pharmaceutical Agents with Crystal Growth Modifiers;" U.S. Patent No. 5,560,932, for "Microprecipitation of Nanoparticulate Pharmaceutical Agents;" U.S. Patent No. 5,543,133, for "Process of Preparing X-Ray Contrast Compositions Containing Nanoparticles;" U.S. Patent No. 5,534,270, for "Method of Preparing Stable Drug Nanoparticles;" U.S. Patent No. 5,510,118, for "Process of Preparing Therapeutic Compositions Containing Nanoparticles;" and U.S. Patent No. 5,470,583, for "Method of Preparing Nanoparticle Compositions Containing Charged Phospholipids to Reduce Aggregation all of which are specifically incorporated by reference.

If the active agent is to be formulated into a nanoparticulate particle size prior to inclusion in the solid dosage form, and the active agent is to prepared by milling, microfluidization, or another suitable mechanical means, it is preferred that the active agent is poorly soluble in at least one liquid dispersion medium. By "poorly soluble" it is meant that the active agent has a solubility in a liquid dispersion medium of less than about 30 mg/ml, less than about 1.0 mg/ml, or less than about 1 mg/ml. Such a liquid dispersion medium can be, for example, water, aqueous salt solutions, oils such as safflower oil, and solvents such as ethanol, t-butanol, hexane, and glycol,

### 2. Spray Drying of Nanoparticulate Active Agents Dispersions or Micron-sized Active Agent Solutions

Solid dosage forms of nanoparticulate active agent dispersions, can be prepared by drying the liquid formulations. An exemplary drying method is spray drying. The spray drying process is used to obtain a nanoparticulate active agent powder which can be formulated into solid dosage forms for administration.

In an exemplary spray drying process, the active agent dispersion or solution is fed to an atomizer using a peristaltic pump and atomized into a fine spray of droplets. The spray is contacted with hot air in the drying chamber resulting in the evaporation of moisture from the droplets. The resulting spray is passed into a cyclone where the powder is separated and collected The active agent dispersion or solution can be spray-dried in the presence or absence of excipient to give the spray-dried intermediate powder. This powder can then be combined with pullulan by, for example, blending with a pullulan powder, or a pullulan solution can be spray granulated onto the active agent powder. Alternatively, the pullulan can be dissolved in the active agent dispersion or solution prior to spray drying.

### 3. Lyophilization

A fast melt solid dosage form of the invention can be prepared by lyophilization, as described above. Suitable lyophilization conditions include, for example, those described in EP 0,363,365 (McNeil-PPC Inc.), U.S. Patent No. 4,178,695 (A. Erbeia), and U.S. Patent No. 5,384,124 (Farmalyoc), all of which are incorporated herein by reference. Typically, a liquid composition comprising a nanoparticulate or micron-sized active agent and pullulan is placed in a suitable vessel and frozen to a temperature of between about -5°C to about -100°C. The nanoparticulate active agent can additionally comprise one or more surface stabilizers adsorbed to the surface thereof. One or more pharmaceutically acceptable sugars and/or plasticizers can be added to the solid dosage form. The frozen liquid is then subjected to reduced pressure for a period of up to about 48 hours. The combination of parameters such as temperature, pressure, liquid medium, and batch size will impact the time required for the lyophilization process. Under conditions of reduced temperature and pressure, the frozen solvent is removed by sublimation yielding a solid, porous, rapidly disintegrating solid dosage form having the active agent distributed throughout.

### 4. Granulation

Alternatively, a solid dosage form of the invention can be prepared by granulating in a fluidized bed an admixture comprising a liquid of a nanoparticulate or micron-sized active agent and pullulan to form a granulate. The nanoparticulate active agent can additionally comprise one or more surface stabilizers adsorbed to the surface thereof. One or more pharmaceutically acceptable sugars and/or plasticizers can be added to the solid dosage form. This is followed by tableting of the granulate to form a solid dosage form.

### 5. Tableting

The solid dosage forms of the invention can be in the form of tablets for oral administration. Preparation of such tablets can be by pharmaceutical compression or molding techniques known in the art. The tablets of the invention may stake any appropriate shape, such as discoid, round, oval, oblong, cylindrical, triangular, hexagonal, and the like.

Powders for tableting can be formulated into tablets by any method known in the art. Suitable methods include, but are not limited to, milling, fluid bed granulation, dry granulation, direct compression, spheronization, spray congealing, and spray-dying. Detailed descriptions of tableting methods are provided in Remington: The Science and Practice of Pharmacy, 19th ed. Vol. 11 (1995) (Mack Publishing Co., Pennsylvania); and Remington's Pharmaceutical Sciences, Chapter 89, pp. 1633-1658 (Mach Publishing Company, 1990), both of which are specifically incorporated by reference.

In an exemplary process, a solid dosage form can be prepared by blending a nanoparticulate active agent composition with pullulan and optionally, other excipients to form a blend which is then directly compressed into tablets. For example, spray-dried active agent powder can be blended with tablet excipients using a V-blender^{®} (Blend Master Lab Blender, Patterson Kelley Co.) or high-shear mixer, followed by compression of the powder using, for example, an automated Carver press (Carver Laboratory Equipment), single station Korsch^{®} press, or a high-speed Fette^{®} tablet press.

The tablets may be coated or uncoated. If coated they may be sugar-coated (to cover objectionable tastes or odors and to protect against oxidation) or film coated (a thin film of water soluble matter for similar purposes).

### C. Administration of Pullulan-Comprising Solid Dosage Forms

The present invention provides a method of treating a subject, including a mammal or a human, with the solid dosage forms of the invention. The administered pullulan-comprising solid dosage forms comprise fast onset of activity with a low friability.

In general, the compositions of the invention will be administered orally to a subject in need thereof using a level of active agent that is sufficient to provide the desired physiological effect. The subject may be a mammal, such as a domestic animal or pet, but preferably is a human subject. The level of active agent needed to give the desired physiological result is readily determined by one of ordinary skill in the art by referring to standard texts, such as *Goodman and Gillman* and the *Physician's Desk Reference.*

The following examples are given to illustrate the present invention. It should be understood, however, that the invention is not to be limited to the specific conditions or details described in these examples. Throughout the specification, any and all references to a publicly available documents are specifically incorporated into this patent application by reference.

### Example 1

The purpose of this example was to prepare a rapidly dissolving solid dosage form of Compound A comprising pullulan.

A nanoparticulate Compound A dispersion was prepared by first combining 10% (w/w) Compound A and 2.5% polyvinyl pyrrolidone (PVP K29/32) as a surface stabilizer, followed by milling the mixture under high energy milling conditions in a DYNO®-Mill KDL (Willy A. Bachofen AG, Maschinenfabrik, Basel, Switzerland) equipped with a 150 cc batch chamber, utilizing 500 µm polymeric attrition media. Milling was conducted until a final mean particle size of 108 nm for the Compound A particles was achieved. Particle size analysis was performed with a Horiba LA-910 particle size analyzer (Irvine, CA).

A mixture of a pullulan solution and the nanoparticulate Compound A dispersion was prepared by combining a solution of pullulan (0.150 g), mannitol (0.6 g), and water for injection (4.7 g) with 0.45 grams of the nanoparticulate dispersion of crystalline Compound A.

A wafer tray with 2.5 cc wells was filled by placing 2.0 grams of the mixture of the nanoparticulate Compound A/pullulan solution into each well. The tray was then lyophilized for 48 hours. After lyophilization, the wafers showed good physical composition and could be handled without breaking. The wafers disintegrated within a few seconds when placed in approximately 5 cc of water. Measurement in a Horiba LA-910 revealed a mean particle size of 136 nm for the Compound A particles in the reconstituted Compound A dispersion.

This example demonstrates that solid dosage forms of nanoparticulate compositions comprising pullulan can be made, that such dosage forms have remarkably short disintegration times and-low friability (*i.e.*, the tablets could be handled without breaking), and that upon reconstitution the nanoparticulate active agent substantially redisperses to the particle size present prior to incorporation of the active agent into a solid dosage form. This latter point is significant, as if the nanoparticulate active agent does not substantially redisperse, then the dosage form will lose the benefits accorded by formulating the active agent into a nanoparticulate size; *i.e.*, greater bioavailability, faster onset of activity, etc.

### Example 2

The purpose of this example was to prepare a rapidly dissolving solid dosage form of Compound B comprising pullulan.

A nanoparticulate dispersion of Compound B was prepared by combining 25% (w/w) Compound B, 5% hydroxypropyl cellulose (HPC-SL), and 0.25% docusate sodium, following by milling the mixture under high energy milling conditions in a DYNO®-Mill KDL (Willy A. Bachofen AG, Maschinenfabrik, Basel, Switzerland) equipped with a 300 cc recirculation chamber, utilizing 500 µm polymeric attrition media, until a final mean particle size of 152 nm for the Compound B particles was achieved. Particle size analysis was performed with a Horiba LA-910 particle size analyzer (Irvine, CA).

A solution of pullulan (0.375 g), mannitol (0.375g), glycerol (0.05 g), and water for injection (7.2 g) was prepared. Next, 2.0 grams of the nanoparticulate Compound B dispersion was added to the pullulan solution.

A wafer tray with 2.5 cc wells was filled by placing 2.0 grams of the mixture of the nanoparticulate Compound B dispersion/pullulan solution into each well. The tray was then lyophilized for 48 hours.

After lyophilization, the wafers showed good physical composition and could be handled without breaking. The wafers disintegrated within 1 minute when placed in approximately 10 cc of water. Measurement in a Horiba LA-910 revealed a mean particle size of 169 nm for the Compound B particles in the reconstituted Compound B dispersion.

This example demonstrates that solid dosage forms of nanoparticulate compositions comprising pullulan can be made, that such dosage forms have remarkably short disintegration times and low friability (*i.e.*, the tablets could be handled without breaking), and that upon reconstitution the nanoparticulate active agent substantially redisperses to the particle size present prior to incorporation of the active agent into a solid dosage form.

### Example 3

The purpose of this example was to prepare a rapidly dissolving solid dosage form of cyclosporin comprising pullulan. Cyclosporin (Sandimmune®, Neoral®, SangCya®) is used to prevent organ rejection after transplant. It has also been used to treat other illnesses, such as aplastic anemia, or to prevent graft versus host disease (GVHD).

A nanoparticulate dispersion of cyclosporin was made by combining 15% (w/w) cyclosporin, 4.15% HPC-SL, and 0.225% docusate sodium, followed by milling the mixture under high energy milling conditions in a DYNO®-Mill KDL (Willy A. Bachofen AG, Maschinenfabrik, Basel, Switzerland) equipped with a 300 cc recirculation chamber, utilizing 500 µm polymeric attrition media, until a final mean particle size of 200 nm for the cyclosporin particles was achieved. Particle size analysis was performed with a Horiba LA-910 particle size analyzer (Irvine, CA).

A solution of pullulan (20.0 g), mannitol (40.0 g), glycerol (10.0 g), and water for injection (596.7 g) was prepared, and 133.3 grams of the nanoparticulate cyclosporin dispersion was added to the pullulan solution.

A wafer tray with 2.5 cc wells was filled by placing 2.0 grams of the mixture of the nanoparticulate cyclosporin dispersion/pullulan solution into each well. The tray was then lyophilized for 48 hours.

After lyophilization, the wafers showed good physical composition and could be handled without breaking. The wafers disintegrated within a few seconds when placed in approximately 5 cc of water. Measurement in a Horiba LA-910 revealed a mean particle size of 258 nm for the cyclosporin particles in the reconstituted cyclosporin dispersion.

This example demonstrates that solid dosage forms of nanoparticulate compositions comprising pullulan can be made, that such dosage forms have remarkably short disintegration times and low friability (*i.e*., the tablets could be handled without breaking), and that upon reconstitution the nanoparticulate active agent substantially redisperses to the particle size present prior to incorporation of the active agent into a solid dosage form.

### Example 4

The purpose of this example was to prepare a rapidly dissolving solid dosage form of Compound C and pullulan.

A nanoparticulate Compound C dispersion was prepared by combining 25% (w/w) Compound C and 8% lysozyme. The NCD was milled under high energy milling conditions in a DYNO®-Mill KDL (Willy A. Bachofen AG, Maschinenfabrik, Basel, Switzerland), utilizing 500 µm polymeric attrition media, until a final mean particle size of 116 nm for the Compound C particles was achieved. Particle size analysis was performed with a Horiba LA-910 particle size analyzer (Irvine, CA).

A solution of pullulan (0.5 g), mannitol (0.5 g), and water for injection (7.0 g) was prepared, and 2.0 grams of the nanoparticulate Compound C dispersion was added to the pullulan solution.

A wafer tray with 2.5 cc wells was filled by placing 2.0 grams of the mixture of the nanoparticulate Compound C dispersion/pullulan solution into each well. The tray was then lyophilized for 48 hours.

After lyophilization, the wafers showed good physical composition and could be handled without breaking. The wafers disintegrated within 10 seconds when placed in approximately 15 cc of water. The reconstituted particle size was 155 nm.

This example demonstrates that solid dosage forms of nanoparticulate compositions comprising pullulan can be made, and that such dosage forms have remarkably short disintegration times and low friability (*i.e.*, the tablets could be handled without breaking).

### Example 5

The purpose of this example was to prepare a rapidly dissolving solid dosage form of Compound D comprising pullulan.

A nanoparticulate Compound D dispersion was prepared by combining 10% (w/w) Compound D and 2% HPC-SL, followed by milling the mixture under high energy milling conditions in a DYNO®-Mill KDL (Willy A. Bachofen AG, Maschinenfabrik, Basel, Switzerland) equipped with a 150 cc batch chamber, utilizing 500 µm polymeric attrition media. Particle size analysis was performed with light microscopy due to the high solubility of the drug. The light microscope showed small, well dispersed particles.

The 10% (w/w) Compound D dispersion was diluted post-milling with sterile water for injection making 38 grams of 5% (w/w) Compound D-dispersion. Pullulan (1.9 g) and mannitol (3.8 g) were added to the 38 grams of nanoparticulate Compound D dispersion. Glycerin (0.12 g) was added to 8 grams of the pullulan, mannitol and Compound D dispersion.

A wafer tray with 2.5 cc wells was filled by placing 2.0 grams of the mixture of the nanoparticulate Compound D dispersion/pullulan solution into each well. Each wafer contained the following: 0.100 g of pullulan, 0.200 g of mannitol, 0.030 g of glycerol, and 0.100 g Compound D. The tray was then lyophilized for 48 hours.

After lyophilization, the wafers showed good physical composition and could be handled without breaking. The wafers disintegrated within 3 minutes when placed in 40 cc of water. Upon reconstitution in water the drug particles dissolved completely.

This example demonstrates that solid dosage forms of nanoparticulate compositions comprising pullulan can be made, and that such dosage forms have remarkably short disintegration times and low friability (*i.e*., the tablets could be handled without breaking).

### Example 6

The purpose of this example was to prepare a rapidly dissolving solid dosage form of Compound D free base comprising pullulan.

A nanoparticulate dispersion of Compound D free base was prepared by combining 5% (w/w) Compound D free base, 1% hydroxypropylmethyl cellulose (HPMC), and 0.05% docusate sodium, followed by milling the mixture under high energy milling conditions in a DYNO®-Mill KDL (Willy A. Bachofen AG, Maschinenfabrik, Basel, Switzerland) equipped with a 150 cc batch chamber, utilizing 500 µm polymeric attrition media, until a final mean particle size of 258 nm for the Compound D particles was achieved. Particle size analysis was performed with a Horiba LA-910 particle size analyzer (Irvine, CA).

Pullulan (0.8 g) and mannitol, (1.6 g) was added to 16 grams of nanoparticulate Compound D free base dispersion. Glycerin (0.06 g) was added to 8 grams of the pullulan, mannitol and Compound D free base dispersion.

A wafer tray with 2.5 cc wells was filled by placing 2.0 grams of the mixture of the Compound D free base nanoparticulate dispersion/pullulan solution into each well. The tray was then lyophilized for 48 hours. Each wafer contained the following: 0.100 g ofpullulan, 0.200 g of mannitol, 0.015 g of glycerol, and 0.100 g Compound D free base.

After lyophilization, the wafers showed good physical composition and could be handled without breaking. The wafers disintegrated within 3.5 minutes when placed in 40 cc of water. Measurement in a Horiba LA-910 revealed a mean particle size of 268 nm for the Compound D particles in the reconstituted Compound D dispersion.

This example demonstrates that solid dosage forms of nanoparticulate compositions comprising pullulan can be made, that such dosage forms have remarkably short disintegration times and low friability (*i.e*., the tablets could be handled without breaking), and that upon reconstitution the nanoparticulate active agent substantially redisperses to the particle size present prior to incorporation of the active agent into a solid dosage form.

### Example 7

The purpose of this example was to test the friability of nanoparticulate Compound C fast melt wafers comprising pullulan.

Friability measures the "robustness" of a dosage form. This is significant as dosage forms having high friability are difficult to package, and have increased manufacturing costs. Conventional fast melt dosage formulations tend to have a high friability.

A nanoparticulate Compound C dispersion was prepared by combining 25% (w/w) Compound C, 5% polyvinyl pyrrolidone (PVP K29/32), and 0.1% docusate sodium. The NCD was milled under high energy milling conditions in a DYNO®-Mill KDL (Willy A. Bachofen AG, Maschinenfabrik, Basel, Switzerland), utilizing 500 µm polymeric attrition media, until a final mean particle size of 354 nm was achieved. Particle size analysis was performed with a Horiba LA-910 particle size analyzer (Irvine, CA).

A solution of pullulan (3.0 g), mannitol (3.0 g), and water for injection (42.0 g) was prepared, and 12.0 grams of the nanoparticulate Compound C dispersion was added to the pullulan solution. After lyophilization, 14 of the wafers were tested in a friabilator (VanKel model 45-2000). The wafers had an initial weight of 4.5197 grams. The wafers were tumbled for 100 drops, removed individually, and any dust was blown off. None of the wafers were fractured and the final weight was 4.4940 grams.

The United States Pharmacopeia (USP) tablet friability test requires no broken tablets and a weight loss of < 1 %. As no broken tablets were observed, and the weight loss was only 0.6%, the Compound C fast melt wafers meet the USP tablet friability requirements.

### Example 8

The purpose of this example was to prepare a solid dose form of naproxen comprising pullulan.

A nanoparticulate naproxen dispersion,was prepared as follows. An aqueous slurry of 30% (w/w) naproxen and 7.5% lysozyme as a surface stabilizer was milled under high energy milling conditions in a NanoMill-2 system (Elan Drug Delivery) equipped with a 20-liter recirculation vessel and utilizing 500 µm polymeric - attrition media. The mean naproxen particle size following milling was 96 nm, with D90 of 139 nm. Particle size analysis was performed with a Horiba LA-910 particle size analyzer (Irvine, CA).

A solution of pullulan (1.8 g), mannitol (6.0 g), glycerol (1.2 g), and water for injection (31.0 g) was prepared and 20.0 grams of a nanoparticulate dispersion of naproxen was added. The NCD contained 30% (w/w) naproxen and 7.5% lysozyme as a surface stabilizer. The NCD was milled under high energy milling conditions in a NanoMill-2 system equipped with a 20-liter recirculation vessel and utilizing 500 µm polymeric attrition media. The particle size before lyophilization was 96 nm. Particle size analysis was performed with a Horiba LA-910 particle size analyzer (Irvine, CA).

A wafer tray with 2.5 cc wells was filled by placing 2.0 grams of the NCD/Pullulan mixture into each well. The tray was then lyophilized for 48 hours. After lyophilization, the wafers showed good physical composition and could be handled without breaking. The wafers disintegrated within a few seconds when placed in approximately 5cc of water. A mean naproxen particle size of 118 nm was measured for the reconstituted wafer, with a D90 of 160 nm.

## Claims

1. A solid dosage form Comprising:
(a) at least one active agent that has an effective average particle size, prior to inclusion in the dosage form, of less than about 2 microns; and
(b) pullulan;
wherein the solid dosage form has a friability of less than about 1%.

2. The solid dosage form of claim 1 having a friability selected from the group consisting of less than about 1%, less than about 0.9%, less than about 0.8%, less than about 0.7%, less than about 0.6%, less than about 0.5°/a, less than about 0.4%, less than about 0.3%., and less than about 0.2%

3. The solid dosage form of claim 1 or claim 2, further Comprising:
(a) at least one pharmaceutically acceptable sugar; and/or
(b) at least one pharmaceutically acceptable plasticizer; and/or
(c) at least one effervescent agent.

4. The solid dosage form of claim 3, wherein:
(a) said sugar is selected from the group consisting of sucrose, xylitol, lactose, mannitol, sorbitol, glucose, mannose, fructose, and trehalose; and/or
(b) said plasticizer is glycerin, polyethylene glycol, propylene glycol, or sorbitol.

5. The solid dosage form of claim 3 or claim 4, wherein the concentration of the one or more pharmaceutically acceptable:
(a) sugars can vary from about 1% to about 99% (w/w), based on the total weight of the dry composition; and/or
(b) plasticizers can vary from about 0.01% to about 70% (w/w), based on the total weight of the dry composition.

6. The solid dosage form of any one of claims 1-5, wherein said composition has been lyophilized.

7. The solid dosage form of any one of claims 1-6, wherein said dosage form is selected from the group consisting of controlled release formulations, fast melt formulations, aerosol formulations, lyophilized formulations, tablets, solid lozenges, capsules, and powders.

8. The solid dosage form of claim 7, wherein said dosage form is a fast melt dosage form which substantially completely disintegrates or dissolves upon contact with saliva in a time period selected from the group consisting of less than about 4 minutes, less than about 3.5 minutes, less than about 3 minutes, less than about 2.5 minutes, less than about 2 minutes, less than about 90 seconds, less than about 60 seconds, less than about 45 seconds, less than about 30 seconds, less than about 20 seconds, less than about 15 seconds, less than about 10 seconds, and less than about 5 seconds.

9. The solid dosage form of any one of claims 1-8, wherein said active agent;
(a) is water-soluble or poorly water-soluble; and/or
(b) has highly toxic and/or highly potent properties; and/or
(c) is in the form of crystalline particles, semi-crystalline particles, amorphous particles, semi-amorphous particles, or a mixture thereof.

10. The solid dosage form of any one of claims 1-9, wherein said active agent has an effective average particle size, prior to inclusion in the dosage form, selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

11. The solid dosage form of any one of claims 1-10, wherein the at least one active agent is selected from the group consisting of COX-2 inhibitors, anticancer agents, NSAIDS, proteins, peptides, nutraceuticals, anti-obesity agents, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products and substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio-pharmaceuticals, sex hormones, anti-allergic agents, stimulants and anoretics, sympathomimetics, thyroid agents, vasodilators, xanthines, acne medication, alpha-hydroxy formulations, cystic-fibrosis therapies, asthma therapies, emphysema therapies, respiratory distress syndrome therapies, chronic bronchitis therapies, chronic obstructive pulmonary disease therapies, organ-transplant rejection therapies, therapies for tuberculosis and other infections of the lung, and respiratory illness therapies associated with acquired immune deficiency syndrome.

12. The solid dosage form of claim 11, wherein the nutraceutical is selected from the group consisting of dietary supplements, vitamins, minerals, herbs, healing foods that have medical or pharmaceutical effects on the body, folic acid, fatty acids, fruit and vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish and marine animal oils, and probiotics.

13. The solid dosage form of any one of claims 1 to 12, further comprising at least one surface stabilizer, which is adsorbed to or associated with the surface of the active agent prior to inclusion in the dosage form.

14. The solid dosage form of any one of claims 1-13, wherein the concentration of
(a) pullulan is selected from the group consisting of about 99.9% to about 0.1% (w/w), about 85% to about 1% (w/w), about 60% to about 5% (w/w), and about 30% to about 10% by weight based on the total weight of the dry composition; and/or
(b) the active agent is selected from the group consisting of from about 99.9% to about 0.01%, from about 99.5% to about 0.001%, from about 95% to about 0.1%, and from about 90% to about 0.5% (w/w), by weight based on the total weight of the dry composition, not including other excipients; and/or
(c) the at least one surface stabilizer is selected from the group consisting from about 0.0001% to about 99.9%, from about 5% to about 90%, and front, about 10% to about 70%, by weight, based on the total combined dry weight of the at least one active agent and at least one surface stabilizer, not including other excipients.

15. The solid dosage form of claim 13 or 14, wherein the at least one surface stabilizer is selected from the group consisting of a nonionic surface stabilizer, an anionic surface stabilizer, a cationic surface stabilizer, and an ionic surface stabilizer.

16. The solid dosage form of any one of claims 13 to 15, wherein the at least one surface stabilizer is selected from the group consisting of cetyl pyridinium chloride, gelatin, casein, phosphatides, dextrin, glycerol, gum acacia, cholesterol, tragacanth, stearic acid, stearic acid esters and salts, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers, poloxamines, a charged phospholipid, dimyristoyl phophatidyl glycerol, dioctylsulfosuccinate, dialkyesters of sodium sulfosuccinic acid, sodium lauryl sulfate, alkyl aryl polyether sulfonates, mixtures of sucrose stearate and sucrose distearate, triblock copolymers of the structure: -(-PEO)-(-PBO-)-(-PEO-)-, p-isononylphenoxypoly-(glycidol), decanoyl-N-methylglucamide; n-decyl β-D-glucopyranoside, n-decyl β-D-maltopyranoside, n-dodecyl β-D-glucopyranoside, n-dodecyl β-D-maltoside, heptanoyl-N-methylglucamide, n-heptyl-β-D-glucopyranoside, n-heptyl β-D-thioglucoside, n-hexyl β-D-glucopyranoside, nonanoyl-N-methylglucamide, n-noyl β-D-glucopyranoside, octanoyl-N-methylglucamide, n-octyl-β-D-glucopyranoside, octyl β-D-thioglucopyranoside, lysozyme, a PEG derivatized phospholipid, PEG derivatized cholesterol, a PEG derivatized cholesterol derivative, PEG derivatized vitamin A, PEG derivatized vitamin E, random copolymers of vinyl acetate and vinyl pyrrolidone, cationic polymers, cationic biopolymers, cationic polysaccharides, cationic cellulosics, cationic alginate, cationic nonpolymeric compounds, cationic phospholipids, cationic lipids, benzalkonium chloride, sulfonium compounds, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromide, dodecylbenzyl triethyl ammonium chloride, poly-diallyldimethylammonium chloride (DADMAC), dimethyl ammonium chlorides, alkyldimethylanunonium halogenides, tricetyl methyl ammonium chloride, decyltrimethylammonium bromide, dodecyltriethylammonium bromide, tetradecyltrimethylammonium bromide, methyl trioctylammomnium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL™ ALKAQUA™, alkyl pyridinium salts; amines, amine salts, amine oxides, imide azolinium salts, protonated quaternary acrylamides, methylated quaternary polymers, cationic guar, polymethylmethacrylate trimethylammonium bromide, polyvinylpymolidone-2-dimethylaminoethyl methacrylate dimethyl sulfate, hexadecyltrimethyl ammonium bromide, poly (2-methacryloxyethyltrimethylammonium bromide) (S1001), poly(N-vinylpyrrolidone/2-dimethylaminoethyl methacrylate) di methylsulphate quarternary (S1002), and poly(2-methylacryloxyamidopropyltrimethylammonium chloride) (S1004).

17. The solid dosage form of any one of claims 1-16 comprising one or more pharmaceutically acceptable excipients.

18. Use of a solid dosage form according to any one of claims 1 to 17 for the manufacture of a medicament.

## Patentansprüche

1. Eine feste Dosierungsform, umfassend:
(a) mindestens einen Wirkstoff, der vor Einbringen in die Dosierungsform eine effektive durchschnittliche Partikelgröße von weniger als etwa 2 Mikrometer aufweist, und
(b) Pullulan,
wobei die feste Dosierungsform eine Brüchigkeit von weniger als etwa 1 % aufweist.

2. Feste Dosierungsform nach Anspruch 1 mit einer Brüchigkeit, ausgewählt aus der Gruppe bestehend aus weniger als etwa 1 %, weniger als etwa 0,9 %, weniger als etwa 0,8 %, weniger als etwa 0,7 %, weniger als etwa 0,6 %, weniger als etwa 0,5 %, weniger als etwa 0,4 %, weniger als etwa 0,3 % und weniger als etwa 0,2 %.

3. Feste Dosierungsform nach Anspruch 1 oder 2, ferner umfassend:
(a) mindestens einen pharmazeutisch verträglichen Zucker, und/oder
(b) mindestens einen pharmazeutisch verträglichen Weichmacher, und/oder
(c) mindestens ein Brausemittel.

4. Feste Dosierungsform nach Anspruch 3, wobei:
(a) genannter Zucker ausgewählt ist aus der Gruppe bestehend aus Saccharose, Xylitol, Lactose, Mannitol, Sorbitol, Glucose, Mannose, Fructose und Trehalose, und/oder
(b) es sich bei dem genannten Weichmacher um Glycerin, Polyethylenglycol, Propylenglycol oder Sorbitol handelt.

5. Feste Dosierungsform nach Anspruch 3 oder 4, wobei die Konzentration von einem oder mehreren pharmazeutisch verträglichen:
(a) Zuckern in einem Bereich von etwa 1 Gew.-% bis etwa 99 Gew.-% bezogen auf das Gesamtgewicht der trockenen Zusammensetzung variieren kann, und/oder - 1 -
(b) Weichmachern in einem Bereich von etwa 0,01 Gew.-% bis etwa 70 Gew.-% bezogen auf das Gesamtgewicht der trockenen Zusammensetzung variieren kann.

6. Feste Dosierungsform nach einem der Ansprüche 1-5, wobei die Zusammensetzung lyophilisiert ist.

7. Feste Dosierungsform nach einem der Ansprüche 1-6, wobei die Dosierungsform ausgewählt ist aus der Gruppe bestehend aus Formulierungen mit kontrollierter Freisetzung, schnell schmelzenden Formulierungen, Aerosolformulierungen, lyophilisierten Formulierungen, Tabletten, festen Lutschtabletten, Kapseln und Pulvern.

8. Feste Dosierungsform nach Anspruch 7, wobei es sich bei der genannten Dosierungsform um eine schnell auflösende Dosierungsform handelt, welche bei Kontakt mit Speichel in einem Zeitraum ausgewählt aus der Gruppe bestehend aus weniger als etwa 4 Minuten, weniger als etwa 3,5 Minuten, weniger als etwa 3 Minuten, weniger als etwa 2,5 Minuten, weniger als etwa 2 Minuten, weniger als etwa 90 Sekunden, weniger als etwa 60 Sekunden, weniger als etwa 45 Sekunden, weniger als etwa 30 Sekunden, weniger als etwa 20 Sekunden, weniger als etwa 15 Sekunden, weniger als etwa 10 Sekunden und weniger als etwa 5 Sekunden im Wesentlichen vollständig zerfällt oder sich auflöst.

9. Feste Dosierungsform nach einem der Ansprüche 1-8, wobei der Wirkstoff
(a) wasserlöslich oder schlecht wasserlöslich ist, und/oder
(b) stark toxisch ist und/oder stark wirksame Eigenschaften aufweist, und/oder
(c) in Form von kristallinen Partikeln, halbkristallinen Partikeln, amorphen Partikeln, halbamorphen Partikeln oder einer Mischung daraus vorliegt.

10. Feste Dosierungsform nach einem der Ansprüche 1-9, wobei der Wirkstoff vor dem Einbringen in die Dosierungsform eine effektive durchschnittliche Partikelgröße aufweist, ausgewählt aus der Gruppe bestehend aus Partikelgrößen von weniger als etwa 1900 -2- nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm.

11. Feste Dosierungsform nach einem der Ansprüche 1-10, wobei der mindestens eine Wirkstoff ausgewählt ist aus der Gruppe bestehend aus COX-2-Hemmern, Antikrebsmitteln, nicht-steroidalen Antiphlogistika (NSAIDS, non steroidal anti-inflammatory drugs), Proteinen, Peptiden, Nutrazeutika, Antiadiposita, Kortikosteroiden, Elastasehemmern, Analgetika, Antimykotika, Krebstherapeutika, Antiemetika, Analgetika, kardiovaskulären Mitteln, Antiphlogistika, Antihelmintika, Antiarrhythmika, Antibiotika, Antikoagulantien, Antidepressiva, Antidiabetika, Antiepileptika, Antihistaminika, Antihypertensiva, Antimuskarinika, antimykobakteriellen Mitteln, antineoplastischen Mitteln, Immunsuppressiva, Thyreostatika, Virustatika, Anxiolytika, Sedativa, adstringierenden Mitteln, Beta-adrenozeptor-Antagonisten, Blutprodukten und Blutersatzmitteln, Inotropika, Kontrastmitteln, Antitussiva, diagnostischen Mitteln, Mitteln zur bildgebenden Diagnostik, Diuretika, Dopaminergika, Hämostatika, immunologischen Mitteln, lipidregulierenden Mitteln, Muskelrelaxantien, Parasympathomimetika, parathyroidalem Calcitonin und Biphosphonaten, Prostaglandinen, Radiopharmazeutika, Geschlechtshormonen, Antiallergika, Stimulantien und Anorektika, Sympathomimetika, Schilddrüsenmitteln, Vasodilatatoren, Xanthinen, Aknemitteln, Alphahydroxyformulierungen, Mukoviszidose-Therapeutika, Asthmatherapeutika, Therapeutika gegen Emphysem, Therapeutika gegen akutes Atemnotsyndrom, Therapeutika gegen chronischer Bronchitis, Therapeutika gegen chronisch-obstruktive Lungenkrankheit, Therapeutika zur Verhinderung von Abstoßungsreaktionen nach Organtransplantation, Therapeutika gegen Tuberkulose und andere Infektionen der -3-Lunge, Therapeutika gegen Atemwegserkrankungen in Verbindung mit dem erworbenen Immundefizienzsyndrom (AIDS).

12. Feste Dosierungsform nach Anspruch 11, wobei das Nutrazeutikum ausgewählt ist aus der Gruppe bestehend aus Nahrungsergänzungsmitteln, Vitaminen, Mineralien, Kräutern, Heilnahrungsmitteln, die eine medizinische oder pharmazeutische Wirkung auf den Körper ausüben, Folsäure, Fettsäuren, Obst- und Gemüseextrakten, Vitaminpräparaten, Mineralstoffpräparaten, Phosphatidylserin, Liponsäure, Melatonin, Glucosamin/Chondroitin, Aloe Vera, Guggul, Glutamin, Aminosäuren, grünem Tee, Lycopen, Vollkostprodukten, Nahrungsmittelzusätzen, Kräutern, Phytonährstoffen, Antioxidantien, Flavonoidbestandteilen von Früchten, Nachtkerzenöl, Leinsamen, Fischöl und Ölen von Meerestieren und Probiotika.

13. Feste Dosierungsform nach einem der Ansprüche 1 bis 12, ferner umfassend mindestens einen Oberflächenstabilisator, welcher vor dem Einbringen in die Dosierungsform an der Oberfläche des Wirkstoffs adsorbiert ist oder mit dieser assoziiert ist.

14. Feste Dosierungsform nach einem der Ansprüche 1-13, wobei die Konzentration:
(a) von Pullulan ausgewählt ist aus der Gruppe bestehend aus Konzentrationen von etwa 99,9 Gew.-% bis etwa 0,1 Gew.-%, etwa 85 Gew.-% bis etwa 1 Gew.-%, etwa 60 Gew.-% bis etwa 5 Gew.-%, und etwa 30 Gew.-% bis etwa 10 Gew.-% bezogen auf das Gesamtgewicht der trockenen Zusammensetzung, und/oder
(b) des Wirkstoffs ausgewählt ist aus der Gruppe bestehend aus Konzentrationen von etwa 99,9 Gew.-% bis etwa 0,01 Gew.-%, von etwa 99,5 Gew.-% bis etwa 0,001 Gew.-%, von etwa 95 Gew.-% bis etwa 0,1 Gew.%, und von etwa 90 Gew.-% bis etwa 0,5 Gew.-%, bezogen auf das Gesamtgewicht der trockenen Zusammensetzung, ohne Berücksichtigung anderer Hilfsstoffe, und/oder
(c) des mindestens einen Oberflächenstabilisators ausgewählt ist aus der Gruppe bestehend aus Konzentrationen von etwa 0,0001 Gew.-% bis etwa 99,9 Gew.-%, von -4- etwa 5 Gew.-% bis etwa 90 Gew.-%, und von etwa 10 Gew.-% bis etwa 70 Gew.-% bezogen auf das Gesamtgewicht der Kombination aus dem mindestens einen Wirkstoff und dem mindestens einen Oberflächenstabilisator ohne Berücksichtigung anderer Hilfsstoffe.

15. Feste Dosierungsform nach einem der Ansprüche 13 oder 14, wobei der mindestens eine Oberflächenstabilisator ausgewählt ist aus der Gruppe bestehend aus einem nichtionischen Oberflächenstabilisator, einem anionischen Oberflächenstabilisator, einem kationischen Oberflächenstabilisator und einem ionischen Oberflächenstabilisator.

16. Feste Dosierungsform nach einem der Ansprüche 13 bis 15, wobei der mindestens eine Oberflächenstabilisator ausgewählt ist aus der Gruppe bestehend aus Cetylpyridiniumchlorid, Gelatine, Casein, Phosphatiden, Dextran, Glycerin, Akaziengummi, Cholesterin, Traganth, Stearinsäure, Stearinsäureestern und -salzen, Calciumstearat, Glycerinmonostearat, Cetostearylalkohol, Cetomacrogol-Emulgierwachs, Sorbitanester, Polyoxyethylenalkylether, Polyoxyethylenrizinusölderivaten, Polyoxyethylensorbitanfettsäureestern, Polyethylenglycolen, Dodecyltrimethylammoniumbromid, Polyoxyethylenstearaten, kolloidalem Siliziumdioxid, Phosphaten, Natriumdodecylsulfat, Carboxymethylcellulose-Calcium, Hydroxypropylcellulosen, Hydroxypropylmethylcellulose, Carboxymethylcellulose-Natrium, Methylcellulose, Hydroxyethylcellulose, Hydroxypropylmethyl-Cellulosephthalat, nichtkristalliner Cellulose, Magnesium-Aluminum-Silikat, Triethanolamin, Polyvinylalkohol, Polyvinylpyrrolidon, 4-(1,1,3,3-Tetramethylbutyl)-Phenolpolymer mit Ethylenoxid und Formaldehyd, Poloxameren, Poloxaminen, einem geladenen Phospholipid, Dimyristoylphophatidylglycerin, Dioctylsulfosuccinat, Dialkylestern der Natriumsulfobernsteinsäure, Natriumlaurylsulfat, Alkylarylpolyethersulfonaten, Gemischen von Sucrosestearat und Sucrosedistearat, Triblock-Copolymeren mit der Struktur: -(-PEO)- (-PBO-)-(-PEO-)-, p-Isononylphenoxypoly-(glycidol), Decanoyl-N-methylglucamid, n-Decyl-β-D-Glucopyranosid, n-Decyl-β-D-Maltopyranosid, n-Dodecyl-β-D-Glucopyranosid, n--5-Dodecyl-β-D-Maltosid, Heptanoyl-N-Methylglucamid, n-Heptyl-β-D-Glucopyranosid, n-Heptyl-β-D-Thioglucosid, n-Hexyl-β-D-Glucopyranosid, Nonanoyl-N-methylglucamid, n-Noyl-β-D-Glucopyranosid, Octanoyl-N-Methylglucamid, n-Octyl-β-D-Glucopyranosid, Octyl-β-D-Thioglucopyranosid, Lysozym, einem PEG-derivatisierten Phospholipid, einem PEG-derivatisierten Cholesterin, einem PEG-derivatisierten Cholesterinderivat, PEG-derivatisiertem Vitamin A, PEG-derivatisiertem Vitamin E, statischen Copolymeren aus Vinylacetat und Vinylpyrrolidon, kationischen Polymeren, kationischen Biopolymeren, kationischen Polysacchariden, kationischer Cellulose, kationischem Alginat, kationischen nichtpolymeren Verbindungen, kationischen Phospholipiden, kationischen Lipiden, Benzalkoniumchlorid, Sulfoniumverbindungen, Phosphoniumverbindungen, quartären Ammoniumverbindungen, Benzyl-di(2-chlorethyl) ethylammoniumbromid, Kokostrimethylammoniumchlorid, Kokostrimethylammoniumbromid, Kokosmethyldihydxoxyethylammoniumchlorid, Kokosmethyldihydroxyethylammoniumbromid, Decyltriethylammoniumchlorid, Decyldimethylhydroxyethylammoniumchlorid, Decyldimethylhydroxyethylammoniumchloridbromid, C₁₂₋₁₅-Dimethylhydroxyethylammoniumchlorid, C₁₂₋₁₅-Dimethylhydroxyethylammoniumchlorid-bromid, Kokosdimethylhydroxyethylammoniumchlorid, Kokosdimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethenoxy)₄-ammoniumchlorid, Lauryldimethyl(ethenoxy)₄-ammoniumbromid, N-alkyl (C₁₂-₁₈)dimethylbenzylammoniumchlorid, N-alkyl(C₁₄₋₁₈)dimethyl-benzyl-ammoniumchlorid, N-Tetradecylidmethylbenzylammoniumchloridmonohydrat, Dimethyldidecylammoniumchlorid, N-alkyl und (C₁₂₋₁₄) Dimethyl-1-napthylmethylammoniumchlorid, Trimethylammoniumhalogen, Alkyl-trimethylammoniumsalzen, Dialkyldimethylammoniumsalzen, Lauryltrimethylammoniumchlorid, ethoxyliertem Alkyamidoalkyldialkylammonimumsalz, einem ethoxylierten Trialkylammoniumsalz, Dialkylbenzoldialkylammoniumchlorid, N-didecyldimethylammoniumchlorid, N-tetradecyldimethylbenzylammonium, Chloridmonohydrat, N-alkyl(C₁₂₋₁₄)dimethyl-1-naphthylmethylammoniumchlorid, Dodecyldimethylbenzylammoniumchlorid, Dialkylbenzolalkylammoniumchlorid, Lauryltrimethylammoniumchlorid, -6-Alkylbenzylmethylammoniumchlorid, Alkylbenzyldimethylammoniumbromid, C₁₂-Trimethylammoniumbromiden, C₁₅-Trimethylammoniumbromiden, C₁₇-Trimethylammoniumbromiden, Dodecylbenzyldiethylammoniumchlorid, Polydiallyldimethylammoniumchlorid (DADMAC), Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT 10™, Tetrabutylammoniumbromid, Benzyltrimethylammoniumbromid, Cholinester, Benzalkoniumchlorid, Stearalkoniumchloridverbindungen, Cetylpyridiniumbromid, Cetylpyridiniumchlorid, Halogensalzen von quartären Polyoxyethylalkylaminen, MIRAPOL™, ALKAQUAT™, Alkylpyridiniumsalzen; Aminen, Aminsalzen, Aminoxiden, Imidazoliniumsalzen, protonierten quartären Acrylamiden, methylierten quartären Polymeren, kationischem Guar, Polymethylmethacrylattrimethylammoniumbromid, Polyvinylpyrrolidon-2-dimethylaminoethylmethacrylatdimethylsulfat, Hexadecyltrimethylammoniumbromid, Poly(2-methacryloxyethyltrimethylammoniumbromid) (S1001), Poly(N-vinylpyrrolidon/2-dimethylaminoethylmethacrylat)dimethylsulfat quartär (S1002) und Poly(2-memylacryloxyamidopropyltrimethylammoniumchlorid) (S1004).

17. Feste Dosierungsform nach einem der Ansprüche 1-16, umfassend einen oder mehrere pharmazeutisch verträgliche Hilfsstoffe.

18. Verwendung einer festen Dosierungsform nach einem der Ansprüche 1 bis 17 zur Herstellung eines Medikaments.

## Revendications

1. Forme posologique solide comprenant :
(a) au moins un agent actif qui a une taille moyenne de particule efficace, avant inclusion dans la forme posologique, inférieure à environ 2 microns ; et
(b) du pullulane ;
où la forme posologique solide a une friabilité inférieure à environ 1 %.

2. Forme posologique solide selon la revendication 1, ayant une friabilité choisie dans le groupe consistant en inférieure à environ 1 %, inférieure à environ 0,9 %, inférieure à environ 0,8 %, inférieure à environ 0,7 %, inférieure à environ 0,6 %, inférieure à environ 0,5 %, inférieure à environ 0,4 %, inférieure à environ 0,3 % et inférieure à environ 0,2 %.

3. Forme posologique solide selon la revendication 1 ou la revendication 2, comprenant en outre :
(a) au moins un sucre pharmaceutiquement acceptable ; et/ou
(b) au moins un plastifiant pharmaceutiquement acceptable ; et/ou
(c) au moins un agent effervescent.

4. Forme posologique solide selon la revendication 3, dans laquelle :
(a) ledit sucre est choisi dans le groupe consistant en le saccharose, le xylitol, le lactose, le mannitol, le sorbitol, le glucose, le mannose, le fructose et le tréhalose ; et/ou
(b) ledit plastifiant est la glycérine, le poly(éthylène glycol), le propylène glycol ou le sorbitol.

5. Forme posologique solide selon la revendication 3 ou la revendication 4, dans laquelle la concentration :
(a) des un ou plusieurs sucres pharmaceutiquement acceptables peut varier d'environ 1 % à environ 99 % (p/p), par rapport au poids total de la composition sèche ; et/ou
(b) des un ou plusieurs plastifiants pharmaceutiquement acceptables peut varier d'environ 0,01 % à environ 70 % % (p/p), par rapport au poids total de la composition sèche.

6. Forme posologique solide selon l'une quelconque des revendications 1 à 5, dans laquelle ladite composition a été lyophilisée.

7. Forme posologique solide selon l'une quelconque des revendications 1 à 6, dans laquelle ladite forme posologique est choisie dans le groupe consistant en les formulations à libération régulée, les formulations à fusion rapide, les formulations aérosol, les formulations lyophilisées, les comprimés, les tablettes solides, les gélules et les poudres.

8. Forme posologique solide selon la revendication 7, dans laquelle ladite forme posologique est une forme posologique à fusion rapide qui se désintègre ou se dissout sensiblement complètement lors d'un contact avec la salive dans une durée choisie dans le groupe consistant en moins d'environ 4 minutes, moins d'environ 3,5 minutes, moins d'environ 3 minutes, moins d'environ 2,5 minutes, moins d'environ 2 minutes, moins d'environ 90 secondes, moins d'environ 60 secondes, moins d'environ 45 secondes, moins d'environ 30 secondes, moins d'environ 20 secondes, moins d'environ 15 secondes, moins d'environ 10 secondes et moins d'environ 5 secondes.

9. Forme posologique solide selon l'une quelconque des revendications 1 à 8, dans laquelle ledit agent actif :
(a) est soluble dans l'eau ou médiocrement soluble dans l'eau ; et/ou
(b) a des propriétés hautement toxiques et/ou hautement puissantes ; et/ou
(c) se présente sous la forme de particules cristallines, de particules semi-cristallines, de particules amorphes, de particules semi-amorphes, ou l'un de leurs mélanges.

10. Forme posologique solide selon l'une quelconque des revendications 1 à 9, dans laquelle ledit agent actif a une taille moyenne de particule efficace, avant inclusion dans la forme posologique, choisie dans le groupe consistant en inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 100 nm, inférieure à environ 75 nm et inférieure à environ 50 nm.

11. Forme posologique solide selon l'une quelconque des revendications 1 à 10, dans laquelle le au moins un agent actif est choisi dans le groupe consistant en les inhibiteurs de COX-2, les agents anticancéreux, les AINS, les protéines, les peptides, les nutraceutiques, les agents anti-obésité, les corticostéroïdes, les inhibiteurs d'élastase, les analgésiques, les antifongiques, les traitements d'oncologie, les antiémétiques, les analgésiques, les agents cardiovasculaires, les agents anti-inflammatoires, les anthelminthiques, les agents anti-arythmiques, les antibiotiques, les anticoagulants, les antidépresseurs, les agents antidiabétiques, les antiépileptiques, les antihistaminiques, les agents antihypertenseurs, les agents antimuscariniques, les agents antimycobactériens, les agents antinéoplasiques, les immunosuppresseurs, les agents antithyroïdiens, les agents antiviraux, les anxiolytiques, les sédatifs, les astringents, les agents inhibiteurs des bêta-adrénocepteurs les produits et substituts sanguins, les agents inotropes cardiaques, les milieux de contraste, les antitussifs, les agents de diagnostic, les agents d'imagerie diagnostique, les diurétiques, les dopaminergiques, les hémostatiques, les agents immunologiques, les régulateurs de lipides, les myorelaxants, les substances parasympathomimétiques, la calcitonine parathyroïdienne et les biphosphonates, les prostaglandines, les produits radiopharmaceutiques, les hormones sexuelles, les agents antiallergiques, les stimulants et anorexigènes, les substances sympathomimétiques, les agents thyroïdiens, les vasodilatateurs, les xanthines, une médication contre l'acné, les formulations alpha-hydroxy, les thérapies de la mucoviscidose, les thérapies de l'asthme, les thérapies de l'emphysème, les thérapies du syndrome de détresse respiratoire, les thérapies de la bronchite chronique, les thérapies de la broncho-pneumopathie obstructive chronique, les thérapies du rejet de greffe d'organe, les thérapies pour la tuberculose et d'autres infections du poumon, et les thérapies de maladies respiratoires associées au syndrome d'immunodéficience acquise.

12. Forme posologique solide selon la revendication 11, dans laquelle le nutraceutique est choisi dans le groupe consistant en les compléments alimentaires, les vitamines, les minéraux, les herbes, les aliments curatifs qui ont des effets médicaux ou pharmaceutiques sur le corps, l'acide folique, les acides gras, les extraits de fruits et légumes, les compléments de vitamine, les compléments de minéraux, la phosphatylsérine, l'acide lipoïque, la mélatonine, la glucosamine/chondroïtine, l'Aloe Vera, le guggul, la glutamine, les acides aminés, le thé vert, le lycopène, les aliments complets, les additifs alimentaires, les herbes, les phytonutriments, les antioxydants, les constituants flavonoïdes des fruits, l'huile d'onagre, les graines de lin, les huiles de poissons et d'animaux marins, et les probiotiques.

13. Forme posologique solide selon l'une quelconque des revendications 1 à 12, comprenant en outre au moins un stabilisant de surface, qui est adsorbé sur ou associé à la surface de l'agent actif avant inclusion dans la forme posologique.

14. Forme posologique solide selon l'une quelconque des revendications 1 à 13, dans laquelle la concentration :
(a) du pullulane est choisie dans le groupe consistant en environ 99,99 % à environ 0,1 % (p/p), environ 85 % à environ 1 % (p/p), environ 60 % à environ 5 % (p/p) et environ 30 % à environ 10 % (p/p) en poids par rapport au poids total de la composition sèche ; et/ou
(b) de l'agent actif est choisie dans le groupe consistant en environ 99,9 % à environ 0,01 %, environ 99,5 % à environ 0,001 %, environ 95 % à environ 0,1 % et environ 90 % à environ 0,5 % (p/p), en poids par rapport au poids total de la composition sèche, n'incluant pas d'autres excipients ; et/ou
(c) du au moins un stabilisant de surface est choisie dans le groupe consistant en environ 0,0001 % à environ 99,9 %, environ 5 % à environ 90 % et environ 10 % à environ 70 %, en poids, par rapport au poids sec combiné total de l'au moins un agent actif et du au moins un stabilisant de surface, n'incluant pas d'autres excipients.

15. Forme posologique solide selon la revendication 13 ou 14, dans laquelle le au moins un stabilisant de surface est choisi dans le groupe consistant en un stabilisant de surface non ionique, un stabilisant de surface anionique, un stabilisant de surface cationique et un stabilisant de surface ionique.

16. Forme posologique solide selon l'une quelconque des revendications 13 à 15, dans laquelle le au moins un stabilisant de surface est choisi dans le groupe consistant en le chlorure de cétylpyridinium, la gélatine, la caséine, les phosphatides, le dextrane, le glycérol, la gomme arabique, le cholestérol, la gomme adragante, l'acide stéarique, les esters et sels d'acide stéarique, le stéarate de calcium, le monostéarate de glycérol, l'alcool cétostéarylique, la cire émulsifiante de cétomacrogol, les esters de sorbitan, les alkyléthers polyoxyéthylénés, les dérivés d'huile de ricin polyoxyéthylénés, les esters d'acide gras de sorbitan polyoxyéthylénés, les poly(éthylène glycols), le bromure de dodécyltriméthylammonium, les stéarates polyoxyéthylénés, le dioxyde de silicium colloïdal, les phosphates, le dodécylsulfate de sodium, la carboxyméthylcellulose de calcium, les hydroxypropylcelluloses, l'hydroxypropylméthylcellulose, la carboxyméthylcellulose de sodium, la méthylcellulose, l'hydroxyéthylcellulose, le phtalate d'hydroxypropylméthyl-cellulose, la cellulose non cristalline, le silicate de magnésium aluminium, la triéthanolamine, le poly(alcool vinylique), la poly(vinylpyrrolidone), le polymère de 4-(1,1,3,3-tétraméthylbutyl)-phénol avec l'oxyde d'éthylène et le formaldéhyde, les poloxamères, les poloxamines, un phospholipide chargé, le dimyristoyl phosphatidyl glycérol, le sulfosuccinate de dioctyle, les esters de dialkyle d'acide sulfosuccinique de sodium, le laurylsulfate de sodium, les sulfonates d'alkylaryle polyéther, les mélanges de stéarate de saccharose et de distéarate de saccharose, les copolymères triséquencés de structure : -(-PEO-)-(PBO)-(-PEO-)-, le p-isononylphénoxypoly-(glycidol), le décanoyl-N-méthylglucamide ; le n-décyl β-D-glucopyranoside, le n-décyl β-D-maltopyranoside, le n-dodécyl β-D-glucopyranoside, le n-dodécyl β-D-maltoside, l'heptanoyl-N-méthylglucanide, le n-heptyl-β-D-glucopyranoside, le n-heptyl-β-D-thioglucoside, le n-hexyl β-D-glucopyranoside, le nonanoyl-N-méthylglucamide, le n-noyl β-D-glucopyranoside, l'octanoyl-N-méthylglucamide, le n-octyl-β-D-glucopyranoside, l'octyl β-D-thioglucopyranoside, une lysozyme, un phospholipide PEG-dérivatisé, le cholestérol PEG-dérivatisé, un dérivé de cholestérol PEG-dérivatisé, la vitamine A PEG-dérivatisé, la vitamine E PEG-dérivatisé, les copolymères statistiques d'acétate de vinyle et de vinylpyrrolidone, les polymères cationiques, les biopolymères cationiques, les polysaccharides cationiques, les cellulosiques cationiques, l'alginate cationique, les composés non polymères cationiques, les phospholipides cationiques, les lipides cationiques, le chlorure de benzalkonium, les composés sulfonium, les composés phosphonium, les composés ammonium quaternaire, le bromure de benzyl-di(2-chloroéthyl)éthylammonium, le chlorure de coprah triméthylammonium, le bromure de coprah triméthylammonium, le chlorure de coprah méthyldihydroxyéthylammonium, le bromure de coprah méthyldihydroxyéthylammonium, le chlorure de décyltriéthylammonium, le chlorure de décyldiméthylhydroxyéthylammonium, le chlorure bromure de décyldiméthylhydroxyéthylammonium, le chlorure de C₁₂₋₁₅diméthylhydroxyéthylammonium, le chlorure bromure de C₁₂₋₁₅diméthylhydroxyéthylammonium, le chlorure de coprah diméthylhydroxyéthylammonium, le bromure de coprah diméthylhydroxyéthylammonium, le méthylsulfate de myristyltriméthylammonium, le chlorure de lauryldiméthylbenzylammonium, le bromure de lauryldiméthylbenzylammonium, le chlorure de lauryldiméthyl(éthénoxy)₄ammonium, le bromure de lauryldiméthyl(éthénoxy)4ₐmmonium, un chlorure de N-alkyl (C₁₂₋₁₈) diméthylbenzylammonium, un chlorure de N-alkyl (C₁₄₋₁₈) diméthylbenzylammonium, le chlorure de N-tétradécyldiméthylbenzylammonium monohydrate, le chlorure de diméthyldidécylammonium, un chlorure de N-alkyl et (C₁₂₋₁₄)diméthyl 1-naphtylméthylammonium, un halogénure de triméthylammonium, les sels d'alkyl-triméthylammonium, les sels de dialkyl-diméthylammonium, le chlorure de lauryltriméthylammonium, un sel d'alkylamidoalkyldialkylammonium éthoxylé, un sel de trialkylammonium éthoxylé, le chlorure de dialkylbenzènedialkylammonium, le chlorure de N-didécyldiméthylammonium, le chlorure de N-tétradécyldiméthylbenzylammonium monohydrate, un chlorure de N-alkyl (C₁₂₋₁₄) diméthyl 1-naphtylméthylammonium, le chlorure de dodécyldiméthylbenzylammonium, un chlorure de dialkylbenzènealkylammonium, le chlorure de lauryltriméthylammonium, un chlorure d'alkylbenzylméthylammonium, un bromure d'alkylbenzyldiméthylammonium, les bromures de C₁₂ triméthylammonium, les bromures de C₁₅ triméthylammonium, les bromures de C₁₇ triméthylammonium, le chlorure de dodécylbenzyltriéthylammonium, le poly(chlorure de diallyldiméthylammonium) (DADMAC), les chlorures de diméthylammonium, les halogénures d'alkyldiméthylammonium, le chlorure de tricétylméthylammonium, le bromure de décyltriméthylammonium, le bromure de dodécyltriéthylammonium, le bromure de tétradécyltriméthylammonium, le chlorure de méthyltrioctylammonium, le POLYQUAT 10^{™}, le bromure de tétrabutylammonium, le bromure de benzyltriméthylammonium, les esters de choline, le chlorure de benzalkonium, les composés chlorure de stéaralkonium, le bromure de cétylpyridinium, le chlorure de cétylpyridinium, les sels d'halogénure de poly(oxyéthylalkylamines) quaternisées, le MIRAPOL^{™}, l'ALKAQUAT^{™}, les sels d'alkylpyridinium; les amines, les sels d'amine, les oxydes d'amine, les sels d'imidazolinium, les acrylamides quaternaires protonés, les polymères quaternaires méthylés, la gomme de guar cationique, le poly(méthacrylate de méthyle) bromure de triméthylammonium, le poly(vinylpyrrolidone-2-méthacrylate de diméthylaminoéthyl-diméthylsulfate), le bromure d'hexadécyltriméthylammonium, le poly(bromure de 2-méthacryloxyéthyltriméthylammonium) (S1001), le diméthylsulfate quaternaire de poly(N-vinylpyrrolidone/méthacrylate de 2-diméthylaminoéthyle) (S1002) et le poly(chlorure de 2-méthacryloxyamidopropyltriméthylammonium) (S1004).

17. Forme posologique solide selon l'une quelconque des revendications 1 à 16, comprenant un ou plusieurs excipients pharmaceutiquement acceptables.

18. Utilisation d'une forme posologique solide selon l'une quelconque des revendications 1 à 17, pour la fabrication d'un médicament.
